(19) Europäisches Patentamt
European Patent Office
Office européen des brevets

(11) Publication number: 0 091 193 B1

(12) # EUROPEAN PATENT SPECIFICATION

(45) Date of publication of the patent specification: 21.05.86

(51) Int. Cl.⁴: A 61 K 31/64, A 61 K 31/44, A 61 K 31/42 // (A61K31/64, 31:42), (A61K31/64, 31:44)

(21) Application number: 83301044.0

(22) Date of filing: 28.02.83

(54) **Antidiabetic compositions.**

(30) Priority: 01.03.82 US 353782
20.12.82 US 450320

(43) Date of publication of application: 12.10.83 Bulletin 83/41

(45) Publication of the grant of the patent: 21.05.86 Bulletin 86/21

(84) Designated Contracting States: AT BE CH DE FR GB IT LI LU NL SE

(56) References cited:
GB - A - 2 080 803
GB - A - 2 083 810

UNLISTED DRUGS, vol. 24, no. 3, March 1972, page 39, O, Chatham, New Jersey, USA, "Diabiformin"
UNLISTED DRUGS, vol. 28, no. 4, April 1976, page 57, C, Chatham, New Jersey, USA, "Glufagos compositum"

(73) Proprietor: PFIZER INC., 235 East 42nd Street, New York, N.Y. 10017 (US)

(72) Inventor: Morville, Malcolm, 9 Hickory Lane, Waterford Connecticut (US)
Inventor: Page, Michael Garth, 3 Winthrop Court, Waterford Connecticut (US)
Inventor: Schnur, Rodney Caughren, 91 Front Street, Noank Connecticut (US)

(74) Representative: Graham, Philip Colin Christison et al, Pfizer Limited Ramsgate Road, Sandwich, Kent CT13 9NJ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 091 193 B1

LIBER, STOCKHOLM 1986

## Description

This invention relates to new pharmaceutical compositions which are of therapeutic value in the treatment of diabetes. More particularly, it is concerned with the concomitant use of two different chemical compounds each chosen from a different class of known organic hypoglycemic agents, but whose concomitant use in this connection has not been previously known.

One of the two known groups of chemical compounds is a series of 5-substituted-oxazolidine-2,4-dione compounds (A) disclosed in BE-A-889757 and BE-A-889758 and U.K. published Patent Applications GB-A-2080803 and GB-A-2083810. This group includes such compounds as 5-(2-methoxy-6-fluorophenyl)oxazolidine-2,4-dione, 5-(2-methoxy-6-chlorophenyl)oxazolidine-2,4-dione, 5-(3-thienyl)-oxazolidine-2,4-dione, 5-(4-ethoxy-3-thienyl)oxa-zolidine-2,4-dione and 5-(2-ethoxy-5-chloro-3-pyridyl)-oxazolidine-2,4-dione, respectively. The other known group of compounds useful in this connection involves several known hypoglycemic sulfonylureas (B) that are thoroughly discussed in a review article appearing in the Chemical & Engineering News, Vol. 59, No. 9, p. 30 (1981) dealing with clinically useful oral antidiabetic agents. Included among this group are such compounds as chlorpropamide, tolbutamide, glipizide and glibenclamide.

According to the present invention, there are provided new oral antidiabetic compositions comprising a compound (A) of the formula:

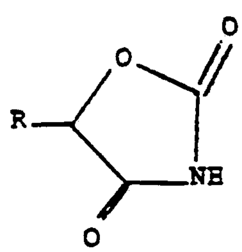

(I)

wherein R is selected from
3-thienyl, 4-ethoxy-3-thienyl, 2-fluorophenyl,
2-methoxyphenyl, 2-ethoxyphenyl, 2-methyl-5-fluorophenyl,
2-methoxy-5-fluorophenyl, 2-methoxy-6-fluorophenyl,
2-methoxy-5-chlorophenyl, 2-methoxy-6-chlorophenyl,
2-methoxy-5-chloro-3-pyridyl and 2-ethoxy-5-chloro-3-pyridyl, or a pharmaceutically acceptable cationic base salt thereof; and a compound (B) of the class of hypoglycemic sulfonylureas selected from chlorpropamide, tolbutamide, acetohexamide, tolazamide, glipizide and glibenclamide, or a pharmaceutically acceptable cationic base salt thereof, with or without a pharmaceutically acceptable carrier or diluent.

Pharmaceutically acceptable cationic base salts of the compounds of formula (1) include the alkali metal salts (e.g., sodium and potassium), alkaline-earth metal salts (e.g., calcium and magnesium), aluminium salts, ammonium salts and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol), and procaine.

More specifically, it has been found that the concomitant use of a compound (A) of the formula (I) with a compound (B) as previously discussed is particularly and unexpectedly valuable in the treatment of diabetes via the oral route of administration. In view of this unexpected synergism, the concomitant oral use of these two compounds in the treatment of diabetes is much more effective than the use of either compound alone when given by the same route of administration.

Preferably, the compounds are administered orally in a ratio of one part by weight of compound (A) of the formula (I) to 0.02-5.0 parts by weight of compound (B). The preferred weight ratio of compound (A) oxazolidinedione to compound (B) sulfonylurea is in the range of 1.0:0.2 to 1.0:2.0, respectively. Preferred embodiments involve the use of 5-(2-methoxy-6-chloro-phenyl)oxazolidine-2,4-dione (A) in conjunction with either chlorpropamide or glipizide (B); 5-(3-thienyl)-oxazolidine-2,4-dione (A) in conjunction with either chlorpropamide or glipizide (B), 5-(2-ethoxy-5-chloro-3-pyridyl)oxazolidine-2,4-dione (A) in conjunction with either chlorpropamide or glipizide (B); 5-(4-ethoxy-2-thienyl)oxazolidine-2,4-dione (A) in conjunction with either chlorpropamide or glipizide (B); and 5-(2-methoxy-6-fluorophenyl)oxazolidine-2,4-dione (A) in conjunction with either chlorpropamide or glipizide (B).

In general, the herein described compounds (A) and (B) can be administered as oral antidiabetic agents in the dose ratios, by weight, indicated above, viz., from about one part by weight of the free acid of compound (A) to 0.02-5.0 parts by weight of compound (B). In this way, they may be administered simultaneously, i.e., in single or separate tablets, capsules, syrups or other oral dosage forms, or sequentially in separate oral dosage forms.

The daily oral dosage levels of the compounds (A) and (B) will depend on the age and weight of the subject being treated, but (expressed as the non-salt form of each compound) will generally be in the respective ranges of approximately 0.5-25 mg/kg of body weight per day of compound (A) of the formula (I) and from about 0.06-25 mg/kg of body weight of compound (B) of the hyloglycemic sulfonylurea class previously described. Thus, for an average adult (70 kg) subject, from approximately 35-1750 mg of compound (A) oxazolidinedione and from approximately 4.0-1750 mg of compound (B) sulfonylurea will be administered per day in either

a single oral dose or up to four divided doses.

Preferably, the compounds will be administered together in the form of a pharmaceutical composition comprising a compound (A) of the formula (I), or a pharmaceutically acceptable cationic base salt thereof, and a compound (B) of the hypoglycemic sulfonylurea class previously described, or a pharmaceutically acceptable cationic base salt thereof, with the weight ratio (expressed as the non-salt form of each compound) of compound (A) oxazolidinedione to compound (B) sulfonylurea being from 1.0:0.02 up to 1.0:5.0, respectively, and the composition will usually also comprise a pharmaceutically acceptable carrier or diluent. The compositions will necessarily be present in a form suitable for oral administration, e.g., in the form of tablets, capsules, emulsions, syrups or elixirs. Suitable compositions can contain from about 35-250 mg of compound (A) of the formula (I) and from about 7.0-250 mg of compound (B) of the hypoglycemic sulfonylurea class previously described, with the weight ratio of compound (A) oxazolidinedione to compound (B) sulfonylurea being in the range of from 1.0:0.02 to 1.0:5.0, as aforesaid, and preferably in the range of 1.0:0.2 to 1.0:2.0, respectively.

Compositions in forms suitable for oral administration may be prepared by methods which are well known to those skilled in the art. For instance, the novel oral antidiabetic compositions of this invention can be administered in a wide variety of different dosage forms, e.g., they may be combined with various pharmaceutically acceptable inert carriers in the form of tablets, capsules, lozenges, troches, hard candies, powders, aqueous suspensions or emulsions, elixirs or syrups. Such carriers include solid diluents or fillers, sterile aqueous media and various non-toxic organic solvents. Moreover, such oral pharmaceutical compositions can be suitably sweetened and/or flavored by means of various commonlyknown agents. In general, the therapeutically-effective compounds (A) and (B) of this invention are present in such oral dosage forms at concentration levels ranging from about 0.5% to about 90% by weight of the total composition, i.e., in amounts which are sufficient to provide the desired unit dosage.

For oral administration, tablets containing various excipients such as sodium citrate, calcium carbonate and dicalcium phosphate may be employed along with various disintegrants such as starch and preferably potato or tapioca starch, alginic acid and certain complex silicates, together with binding agents such polyvinylpyrrolidone, gelatin and acacia. Additionally, lubricating agents such as magnesium stearate, sodium lauryl sulfate and talc are often very useful for tabletting purposes. Solid compositions of a similar type may also be employed as fillers in soft and hard-filled gelatin capsules; preferred materials in this connection would also include the high molecular weight polyethylene glycols. When aqueous suspensions and/or elixirs are desired for oral administration, the essential active ingredient therein may be combined with various sweetening or flavoring agents, coloring matter or dyes and, if so desired, emulsifying and/or suspending agents as well, together with such diluents as water, ethanol, propylene glycol, glycerin and various like combinations thereof.

**EXAMPLE 1**

The following combinations of 5-(3-thienyl) oxazolidine-2,4-dione (Compound No. 1) in conjunction with chlorpropamide, tolbutamide, glibenclamide or glipizide were respectively tested for hypoglycemic activity in terms of their ability to exhibit enhanced glucose control in groups of intact male albino rats that had been fasted for 18 hours prior to use. No anesthetic was used in this study. After the rats had been fasted, different groups were then treated orally with either water (controls) or the aforesaid oxazolidine-2,4-dione compound (as the sodium salt) at the 25 mg./kg. dose level. Thirty minutes later, the groups of rats were dosed intraperitoneally with either saline alone (controls) or one of the aforesaid sulfonylurea compounds (dissolved as the sodium salt-in 0.9% saline) at a dose level ranging from 0.6 mg./kg. to 25 mg./kg., respectively (see table). Blood samples were then taken from the tail vein of each animal thirty minutes following the intraperitoneal dosing. The samples were immediately diluted 1:10 (by volume) with 1.0% heparin in 0.9% saline. Blood glucose concentrations (mg./dl.) were then determined by adapting the method of W. S. Hoffman [Journal of Biological Chemistry, Vol. 120, p. 51 (1937)] to the Autoanalyzer instrument produced bv Technicon Instruments Corporation of Chauncey, N.Y. On this basis, the following results were obtained in terms of blood glucose levels (with standard error of the mean) at the 0.5-hour mark for each combination listed in the table below:

| Hypoglycemic Combination | Blood Glucose (mg./dl.) |
|---|---|
| Control (water plus 0.9% saline) | 72 ± 3.2 |
| Cpd. No. 1 plus saline (0.9%) | 69 ± 2.7 |
| Water plus chlorpropamide (25 mg./kg.) | 63 ± 1.5 |
| Cpd. No. 1 plus chlorpropamide (25 mg./kg.) | 55 ± 0.9 |
| Water plus tolbutamide (25 mg./kg.) | 57 ± 1.6 |
| Cpd. No. 1 plus tolbutamide (25 mg./kg.) | 48 ± 0.9 |
| Water plus glibenclamide (0.6 mg./kg.) | 60 ± 1.4 |
| Cpd. No. 1 plus glibenclamide (0.6 mg./kg.) | 48 ± 1.3 |
| Water plus glipizide (0.6 mg./kg.) | 68 ± 3.2 |
| Cpd. No. 1 plus glipizide (0.6 mg./kg.) | 55 ± 2.1 |

From the data presented in the above table, it is obvious that combinations of 5-(3-thienyl)oxazolidine-2,4-dione (Cpd. No. 1) and the appropriate sulfonylurea produce better therapeutic effects than either compound alone, as attested to by the statistically significant enhancement of the hypoglycemic activity of the

sulfonylurea in the presence of the specifically selected oxazolidine-2,4-dione compound.

**EXAMPLE 2**

The following combinations of 5-(2-methoxy-6-chloro-phenyl)-oxazolidine-2,4-dione (Compound No. 2) with chlorpropamide, tolbutamide, glibenclamide or glipizide were respectively tested for hypoglycemic activity in terms of their ability to exhibit enhanced blood glucose control in groups of intact male albino rats according to the procedure described in Example 1 for the corresponding 5-(3-thienyl) compound. In this particular case, the oxazolidine-2,4-dione compound (i.e., Compound No. 2) was tested at the 5.0 mg./kg. dose level (orally), while the sulfonylurea compound was thereafter administered in the same manner as before (i.e., intraperitoneally) at a dose level ranging from 0.3 mg./kg. to 25 mg./kg., as indicated in the table below, where the results obtained are again reported in terms of blood glucose levels (with standard error of the mean) at the 0.5-hour mark for each of the combinations so specified:

**Hypoglycemic Combination Blood Glucose (mg./dl.)**

Control (water plus 0.9% saline) 78 ± 4.3

Cpd. No. 2 plus saline (0.9%) 73 ± 1.5

Water plus chlorpropamide (25 mg./kg.) 69 ± 1.4

Cpd. No. 2 plus chlorpropamide (25 mg./kg.) 58 ± 0.8

Water plus tolbutamide (25 mg./kg.) 64 ± 1.1

Cpd. No. 2 plus tolbutamide (25 mg./kg.) 56 ± 3.0

Water plus glibenclamide (0.6 mg./kg.) 63 ± 2.1

Cpd. No. 2 plus glibenclamide (0.6 mg./kg.) 59 ± 0.95

Water plus glipizide (0.3 mg./kg.) 71 ± 3.7

Cpd. No. 2 plus glipizide (0.3 mg./kg.) 64 ± 2.5

From the data presented in the above table, it can be seen that combinations of 5-(2-methoxy-6-chlorophenyl)-oxazolidine-2,4-dione (Cpd. No. 2) and the appropriate sulfonylurea prod ue better therapeutic effects than either compound alone, as attested to by the substantially enhanced hypoglycemic activity of the sulfonylurea in the presence of the specifically selected oxazolidine-2,4-dione compound.

**EXAMPLE 3**

The following oral antidiabetic compositions are prepared by combining the active ingredients listed below (oxazolidinedione plus hypoglycemic sulfonylurea) in the amounts indicated in parenthesis, together with standard pharmaceutical excipients (if so desired):

5-(3-thienyl)oxazolidine-2,4-dione (100 mg.) plus chlorpropamide (250 mg.)

5-(3-thienyl)oxazolidine-2,4-dione (100 mg.) plus chlorpropamide (100 mg.)

5-(3-thienyl)oxazolidine-2,4-dione (250 mg.) plus chlorpropamide (100 mg.)

5-(3-thienyl)oxazolidine-2,4-dione (250 mg.) plus chlorpropamide (250 mg.)

5-(2-methoxy-6-chlorophenyl)oxazolidine-2,4-dione (100 mg.) plus chlorpropamide (250 mg.)

5-(2-methoxy-6-chlorophenyl)oxazolidine-2,4-dione (50 mg.) plus chlorpropamide (250 mg.)

5-(2-methoxy-6-chlorophenyl)oxazolidine-2,4-dione (100 mg.) plus chlorpropamide (100 mg.)

5-(2-methoxy-6-chlorophenyl)oxazolidine-2,4-dione (50 ma.) plus chlorpropamide (100 mg.)

5-(2-ethoxy-5-chloro-3-pyridyl)oxazolidine-2,4-dione (50 mg.) plus chlorpropamide (100 mg.)

5-(2-ethoxy-5-chloro-3-pyridyl)oxazolidine-2,4-dione (100 mg.) plus chlorpropamide (100 mg.)

5-(3-thienyl)oxazolidine-2,4-dione (100 mg.) plus tolbutamide (250 mg.)

5-(3-thienyl)oxazolidine-2,4-dione (100 mg.) plus tolazamide (250 mg.)

5-(3-thienyl)oxazolidine-2,4-dione (100 mg.) plus acetohexamide (250 mg.)

5-(3-thienyl)oxazolidine-2,4-dione (100 mg.) plus glibenclamide (50 mg.)

5-(3-thienyl)oxazolidine-2,4-dione (100 mg.) plus glibenclamide (10 mg.)

5-(2-methoxy-6-chlorophenyl)oxazolidine-2,4-dione (100 mg.) plus glipizide (25 mg.)

5-(2-methoxy-6-chlorophenyl)oxazolidine-2,4-dione (100 mg.) plus glibenclamide (10 mg.)

5-(2-ethoxy-5-chloro-3-pyridyl)oxazolidine-2,4-dione (100 mg.) plus glipizide (25 mg.).

**EXAMPLE 4**

The following combinations of 5-(4-ethoxy-3-thienyl)oxazolidine-2,4-dione (Compound No. 3) with chlorpropamide or glipizide were respectively tested for hypoglycemic activity in terms of their ability to exhibit enhanced blood glucose control in groups of intact male albino rats according to the procedure described in Example 1 for the corresponding 5-(3-thienyl) compound. In this particular case the oxazolidine-2,4-dione compound (i.e., Compound No. 3) was tested at the 10 mg./kg. dose level (orally), while the sulfonylurea compound was thereafter administered in the same manner as before (i.e., intraperitoneally at a dose level ranging from 0.3 mg./kg. to 25 mg./kg., as indicated in the table below, where the results obtained are again reported in terms of blood glucose levels (with standard error of the mean) at the 0.5-hour mark for each of the combinations so specified:

**Hypoglycemic Combination Blood Glucose (mg./dl.)**

Control (water plus 0.9% saline) 67 ± 2.2

Cpd. No. 3 plus saline (0.9%) 62.5 ± 1.2

Water plus chlorpropamide (25 mg./kg.) 62.9 ± 3.1

Cpd. No. 3 plus chlorpropamide (25 mg./kg.) 45.9 ± 3.1

Water plus glipizide (0.3 mg./kg.) 61.2 ± 2.5

Cpd. No. 3 plus glipizide (0.3 mg./kg.) 49.9 ± 2.0

From the data presented in the above table, it can be seen that combinations of 5-(4-ethoxy-3-thienyl) oxazolidine-2,4-dione (Cpd. No. 3) and the appropriate sulfonylurea produce better therapeutic effects than either compound alone, as attested to by the substantially enhanced hypoglycemic activity of the sulfonylurea in the presence of the specifically selected oxazolidine-2,4-dione compound.

**EXAMPLE 5**

The following combination of 5-(2-methoxy-6-fluoro-phenyl)oxazolidine-2,4-dione (Compound No. 4) with chlorpropamide or glipizide were respectively tested for hypoglycemic activity in terms of their ability to exhibit enhanced blood glucose control in groups of intact male albino rats according to the procedure described in Example 1 for the corresponding 5-(3-thienyl) compound. In this particular case, the oxazolidine-2,4-dione compound (i.e., Cpd. No. 4) was tested at the 10 mg./kg. dose level (orally), while the sulfonylurea compound was thereafter administered in the same manner as before (i.e., intraperitoneally) at a dose level ranging from 0.3 mg./kg. to 25 mg./kg., as indicated in the table below, where the results obtained are again reported in terms of blood glucose levels (with standard error of the mean) at the 0.5-hour mark for each of the combinations so specified:

**Hypoglycemic Combination Blood Glucose (mg./dl.)**

Control (water + 0.9% saline) 64.7 ± 2.7

Cpd. No. 4 plus saline (0.9%) 58.8 ± 1.2

Water plus chlorpropamide (25 mg./kg.) 56.6 ± 1.9

Cpd. No. 4 plus chlorpropamide (25 mg./kg.) 45.3 ± 3.0

Water plus glipizide (0.3 mg./kg.) 54.7 ± 2.2

Cpd. No. 4 plus glipizide (0.3 mg./kg.) 43.7 ± 3.0

From the data presented in the above table, it can be seen that combinations of 5-(2-methoxy-6-fluorophenyl)-oxazolidine-2,4-dione (Cpd. No. 4) and the appropriate sulfonylurea produce better therapeutic effects than either compound alone, as attested to by the substantially enhanced hypoglycemic activity of the sulfonylurea in the presence of the specifically selected oxazolidine-2,4-dione compound.

**Claims** for the Contracting States BE CH DE FR GB IT LI LU NL SE

1. An oral antidiabetic composition comprising a compound (A) of the formula:

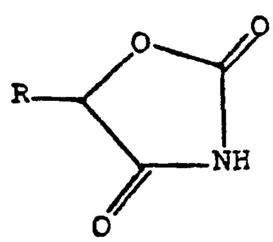

(I)

wherein R is selected from 3-thienyl, 4-ethoxy-3-thienyl, 2-fluorophenyl, 2-methoxyphenyl, 2-ethoxyphenyl, 2-methyl-5-flourophenyl, 2-methoxy-5-fluorophenyl, 2-methoxy-6-fluorophenyl, 2-methoxy-5-chlorophenyl, 2-methoxy-6-chlorophenyl, 2-methoxy-5-chloro-3-pyridyl and 2-ethoxy-5-chloro-3-pyridyl, or a pharmaceutically acceptable cationic base salt thereof; and a compound (B) of the class of hypoglycemic sulfonylureas selected from chlorpropamide, tolbutamide, acetohexamide, tolazamide, glipizide and glibenclamide, or a pharmaceutically acceptable cationic base salt thereof.

2. A composition as claimed in claim 1 wherein compound (A) is an oxazolidinedione of the formula wherein R is 3-thienyl and compound (B) is chlorpropamide or glipizide.

3. A composition as claimed in claim 1 wherein compound (A) is an oxazolidinedione of the formula wherein R is 4-ethoxy-3-thienyl and compound (B) is chlorpropamide or glipizide.

4. A composition as claimed in claim 1 wherein compound (A) is an oxazolidinedione of the formula (I) wherein R is 2-methoxy-6-fluorophenyl and compound (B) is chlorpropamide or glipizide.

5. A composition as claimed in claim 1 wherein compound (A) is an oxazolidinedione of the formula (I) wherein R is 2-methoxy-6-chlorophenyl and compound (B) is chlorpropamide or glipizide.

6. A pharmaceutical composition comprising an oral antidiabetic composition as claimed in any preceding claim, together with a pharmaceutically acceptable carrier or diluent.

7. A composition as claimed in any preceding claim wherein the weight ratio of the compound (A) to the compound (B) is in the range of from 1.0:0.02 to 1.0:5.0.

8. A composition as claimed in claim 7 wherein the ratio is in the range from 1.0:0.2 to 1.0:2.0.

**Claims** for the Contracting State AT

1. A process for preparing an oral diabetic composition which comprises mixing a compound (A) of the formula:

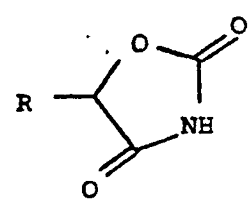

(I)

wherein R is selected from 3-thienyl, 4-ethoxy-3-thienyl, 2-fluorophenyl, 2-methoxyphenyl, 2-ethoxyphenyl, 2-methyl-5-fluorophenyl, 2-methoxy-5-fluorophenyl, 2-methoxy-6-fluorophenyl, 2-methoxy-5-chlorophenyl, 2-methoxy-6-chlorophenyl, 2-methoxy-5-chloro-3-pyridyl or 2-ethoxy-5-chloro-3-pyridyl, or a pharmaceutically acceptable cationic base salt thereof, with a compound (B) of the class of hypoglycemic sulfonylureas selected from chlorpropamide, tolbutamide, acetohexamide, tolazamide, glipizide and glibenclamide, or a pharmaceutically acceptable

cationic base salt thereof, to form a composition in which the compounds (A) and (B) have a synergistic effect in lowering blood sugar when administered orally.

2. A process as claimed in claim 1, in which the compounds (A) and (B) are mixed together with a pharmaceutically acceptable carrier material or diluent.

3. A process as claimed in claim 1 or claim 2 in which the compounds (A) and (B) are mixed in a weight ratio in the range from 1.0:0.02 to 1.0:5.0.

4. A process as claimed in claim 3, in which the ratio is in the range from 1.0:0.2 to 1.0:2.0.

5. A process as claimed in any preceding claim wherein the compound (A) oxazolidinedione is of the formula (I) wherein R is 3-thienyl and the compound (B) sulfonylurea is chlorpropamide or glipizide.

6. A process as claimed in any of claims 1 to 4 wherein the compound (A) oxazolidinedione is of the formula (1) wherein R is 4-ethoxy-3-thienyl and the compound (B) sulfonylurea is chlorpropamide or glipizide.

7. A process as claimed in any of claims 1 to 4 wherein the compound (A) oxazolidinedione is of the formula (1) wherein R is 2-methoxy-6-fluorophenyl and the compound (B) sulfonylurea is chlorpropamide or glipizide.

8. A process as claimed in any of claims 1 to 4 wherein the compound (A) oxazolidinedione is of the formula (I) wherein R is 2-methoxy-6-chlorphenyl and the compound (B) sulfonylurea is chlorpropamide or glipizide.

**Revendications** pour les Etats contractants: BE, CH, DE, FR, GB, IT, LI, LU, NL, SE.

1. Composition antidiabétique orale comprenant un composé (A) de formule :

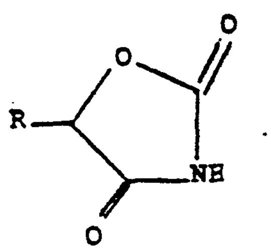

(I)

dans laquelle R est choisi entre les groupes 3-thiényle,

4-éthoxy-3-thiényle, 2-fluorophényle, 2-méthoxyphényle, 2-éthoxyphényle, 2-méthyl-5-fluorophényle, 2-méthoxy-5-fluorophényle, 2-méthoxy-6-fluorophényle, 2-méthoxy-5-chlorophényle, 2-méthoxy-6-chlorophényle, 2-méthoxy-5-chloro-3-pyridyle et 2-éthoxy-5-chloro-3-pyridyle ou un sel de base cationique pharmaceutiquement acceptable de ce composé; et un composé (B) de la classe des sulfonylurées hypoglycémiques, choisi entre le chlorpropamide, le tolbutamide, l'acétohexamide, le tolazamide, le glipizide et le glibenclamide, ou un sel de base cationique pharmaceutiquement acceptable de ce composé.

2. Composition suivant la revendication 1, dans laquelle le composé (A) est une oxazolidinedione de formule (I) dans laquelle R est un groupe 3-thiényle et le composé (B) est le chlorpropamide ou le glipizide.

3. Composition suivant la revendication 1, dans laquelle le composé (A) est une oxazolidinedione de formule (I) dans laquelle R est un groupe 4-éthoxy-3-thiényle et le composé (E) est le chlorpropamide ou le glipizide.

4. Composition suivant la revendication 1, dans laquelle le composé (A) est une oxazolidinedione de formule (I) dans laquelle R est le groupe 2-méthoxy-6-fluorophényle et le composé (B) est le chlorpropamide ou le glipizide.

5. Composition suivant la revendication 1, dans laquelle le composé (A) est une oxazolidinedione de formule (I) dans laquelle R est le groupe 2-méthoxy-6-chlorophényle et le composé (B) est le chlorpropamide ou le glipizide.

6. Composition pharmaceutique comprenant une composition antidiabétique orale suivant l'une quelconque des revendications précédentes, en association avec un support ou diluant pharmaceutiquement acceptable.

7. Composition suivant l'une quelconque des revendications précédentes, dans laquelle le rapport en poids du composé (A) au composé (B) se situe dans l'intervalle de 1,0:0.02 à 1,0:5,0.

8. Composition suivant la revendication 7, dans laquelle le rapport se situe dans l'intervalle de 1,0:0,2 à 1,0:2,0.

**Revendications**

1. Procédé de préparation d'une composition diabétique orale, qui consiste à mélanger un composé (A) de formule :

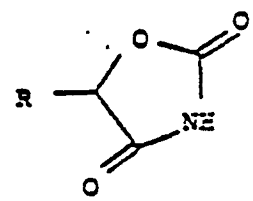

(I)

dans laquelle R est choisi entre les groupes 3-thiényle, 4-éthoxy-3-thiényle, 2-fluorophényle, 2-méthoxyphényle, 2-éthoxyphényle, 2-méthyl-5-fluorophényle, 2-méthoxy-5-fluorophényle, 2-méthoxy-6-fluorophényle, 2-méthoxy-5-chlorophényle, 2-méthoxy-6-chlorophényle, 2-méthoxy-5-chloro-3-pyridyle ou 2-éthoxy-5-chloro-3-pyridyle ou un sel de base cationique pharmaceutiquement acceptable de ce composé, avec un composé (B) de la classe des sulfonylurées hypoglycémiques choisi entre le chlorpropamide, le tolbutamide, l'acétohexamide, le tolazamide, le glipizide et le glibenclamide, ou

un sel de base cationique pharmaceutiquement acceptable de ce compose, pour former une composition dans laquelle les composes (A) et (B) exercent un effet synergique dans l'abaissement du taux sanguin de sucre lorsqu'ils sont administrés oralement.

2. Procédé suivant la revendication 1, dans lequel les composés (A) et (B) sont mélangés avec un support ou diluant pharmaceutiquement acceptable.

3. Procédé suivant la revendication 1 ou la revendication 2, dans lequel les composés (A) et (B) sont mélangés dans un rapport en poids compris dans l'intervalle de 1,0:0,02 à 1,0:5,0.

4. Procédé suivant la revendication 3, dans lequel le rapport se situe dans l'intervalle de 1,0:0,2 à 1,0:2,0.

5. Procédé suivant l'une quelconque des revendications précédentes, dans lequel l'oxazolidine, dione constituant le composé (A) répond à la formule (I) dans laquelle R est le groupe 3-thiényle et la sulfonylurée constituant le composé (B) est le chlorpropamide ou le glipizide.

6. Procédé suivant l'une quelconque des revendications 1 à 4, dans lequel l'oxazolidinedione constituant le composé (A) répond à la formule (I) dans laquelle R est le groupe 4-éthoxy-3-thiényle et la sulfonylurée constituant le composé (B) est le chlorpropamide ou le glipizide.

7. Procédé suivant l'une quelconque des revendications 1 à 4, dans lequel l'oxazolidinedione constituant le composé (A) répond à la formule (I) dans laquelle R est le groupe 2-méthoxy-6-fluorophényle et la sulfonylurée constituant le composé (B) est le chlorpropamide ou le glipizide.

8. Procédé suivant l'une quelconque des revendications 1 à 4, dans lequel l'oxazolidinedione constituant le composé (A) répond à la formule (I) dans laquelle R est le groupe 2-méthoxy-6-chlorophényle et la sulfonylurée constituant le composé (B) est le chlorpropamide ou le glipizide.

**Patentansprüche** BE, CH, DE, FR, GB, IT, LI, LU, NL, SE

1. Orales Antidiabetesmittel, das eine Verbindung (A) Formel

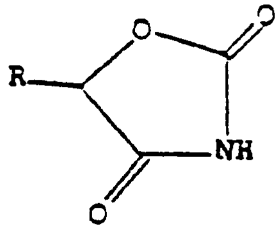

(I),

worin R ausgewählt ist aus 3-Thienyl, 4-Äthoxy-3-thienyl, 2-Fluorphenyl, 2-Methoxyphenyl, 2-Äthoxyphenyl, 2-Methyl-5-fluorphenyl, 2-Methoxy-5-fluorphenyl, 2-Methoxy-6-fluorphenyl, 2-Methoxy-5-chlorphenyl, 2-Methoxy-6-chlorphenyl, 2-Methoxy-5-chlor-3-pyridyl und 2-Äthoxy-5-chlor-3-pyridyl, oder ein pharmazeutisch annehmbares kationisches Basensalz hievon und eine Verbindung (B) der Klasse der hypoglykämischen Sulfonylharnstoffe ausgewählt aus Chlorpropamid, Tolbutamid, Acetohexamid, Tolazamid, Glipizid und Glibenclamid oder ein pharmazeutisch annehmbares kationisches Basensalz hievon umfaßt:

2. Mittel, wie in Anspruch I beansprucht, worin die Verbindung (A) ein Oxazolidindion der Formel (I) ist, worin R 3-Thienyl bedeutet, und die Verbindung (B) Chlorpropamid oder Glipizid ist.

3. Mittel, wie in Anspruch 1 beansprucht, worin die Verbindung (A) ein Oxazolidindion der Formel (I) ist, worin R 4-Äthoxy-3-thienyl bedeutet, und die Verbindung (B) Chlorpropamid oder Glipizid ist.

4. Mittel, wie in Anspruch 1 beansprucht, worin die Verbindung (A) ein Oxazolidindion der Formel (I) ist, worin R 2-Methoxy-6-fluorphenyl bedeutet, und die Verbindung (B) Chlorpropamid oder Glipizid ist.

5. Mittel, wie in Anspruch 1 beansprucht, worin die Verbindung (A) ein Oxazolidindion der Formel (1) ist, worin R 2-Methoxy-6-chlorphenyl bedeutet, und die Verbindung (B) Chlorpropamid oder Glipizid ist.

6. Pharmazeutische Zusammensetzung, die ein orales Antidiabetesmittel, wie in einem der vorhergehenden Ansprüche beansprucht, zusammen mit einem pharmazeutisch annehmbaren Träger oder Verdünnungsmittel umfaßt.

7. Mittel, wie in einem der vorhergehenden Ansprüche beansprucht, worin das Gewichtsverhältnis der Verbindung (A) zur Verbindung (B) im Bereich von 1,0: 0,02 bis 1,0: 5,0 liegt.

8. Mittel, wie in Anspruch 7 beansprucht, worin das Verhältnis im Bereich von 1,0: 0,2 bis 1,0: 2,0 liegt.

**Patentansprüche**

1. Verfahren zum Herstellen eines oralen Diabetesmittels, welches das Mischen einer Verbindung (A) der Formel

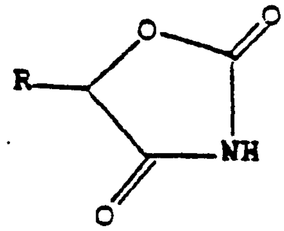

(I),

worin R ausgewählt ist aus 3-Thienyl, 4-Äthoxy-3-thienyl, 2-Fluorphenyl, 2-Methoxyphenyl, 2-Äthoxyphenyl, 2-Methyl-5-fluorphenyl, 2-Methoxy-5-fluorphenyl, 2-Methoxy-6-fluorphenyl, 2-Methoxy-5-chlorphenyl, 2-Methoxy-6-

chlorphenyl, 2-Methoxy-5-chlor-3-pyridyl oder 2-Äthoxy-5-chlor-3-pyridyl, oder eines pharmazeutisch annehmbaren kationischen Basensalzes hievon mit einer Verbindung (B) aus der Klasse der hypoglykämischen Sulfonylharnstoffe ausgewählt aus Chlorpropamid, Tolbutamid, Acetohexamid, Tolazamid, Glipizid und Glibenclamid oder einem pharmazeutisch annehmbaren kationischen Basensalz hievon umfaßt, wobei ein Mittel gebildet wird, in dem die Verbindungen (A) und (B) einen synergistischen Effekt beim Senken von Blutzucker bei oraler Verabreichung aufweisen.

2. Verfahren, wie in Anspruch 1 beansprucht, worin die Verbindungen (A) und (B) mit einem pharmazeutisch annehmbaren Trägermaterial oder Verdünnungsmittel gemischt werden.

3. Verfahren, wie in Anspruch 1 oder Anspruch 2 beansprucht, worin die Verbindungen (A) und (B) in einem Gewichtsverhältnis im Bereich von 1,0: 0,02 bis 1,0: 5,0 gemischt werden.

4. Verfahren, wie in Anspruch 3 beansprucht, worin das Verhältnis im Bereich von 1,0: 0,2 bis 1,0: 2,0 liegt.

5. Verfahren, wie in einem vorhergehenden Anspruch beansprucht, worin die Oxazolidindion-Verbindung (A) die Formel (I) aufweist, worin R 3-Thienyl bedeutet, und die Sulfonylharnstoff-Verbindung (B) Chlorpropamid oder Glipizid ist.

6. Verfahren, wie in einem der Ansprüche 1 bis 4 beansprucht, worin die Oxazolidindion-Verbindung (A) die Formel (I) aufweist, worin R 4-Äthoxy-3-thienyl bedeutet, und die Sulfonylharnstoff-Verbindung (B) Chlorpropamid oder Glipizid ist.

7. Verfahren, wie in einem der Ansprüche 1 bis 4 beansprucht, worin die Oxazolidindion-Verbindung (A) die Formel (I) aufweist, worin R 2-Methoxy-6-fluorphenyl bedeutet, und die Sulfonylharnstoff-Verbindung (B) Chlorpropamid oder Glipizid ist.

8. Verfahren, wie in einem der Ansprüche 1 bis 4 beansprucht, worin die Oxazolidindion-Verbindung (A) die Formel (I) aufweist, worin R 2-Methoxy-6-chlorphenyl bedeutet, und die Sulfonylharnstoff-Verbindung (B) Chlorpropamid oder Glipizid ist.